Europäisches Patentamt

European Patent Office

Office européen des brevets

(19)

(11) Veröffentlichungsnummer: **0 102 033 B2**

(12) # NEUE EUROPÄISCHE PATENTSCHRIFT

(45) Veröffentlichungstag der neuen Patentschrift:
21.08.91 Patentblatt 91/34

(51) Int. Cl.⁵ : **A61B 5/00**

(21) Anmeldenummer: 83108146.8

(22) Anmeldetag: 17.08.83

(54) **Elektrochemischer Sensor zur transcutanen Messung des Kohlendioxid-Partialdrucks eines Lebewesens.**

(30) Priorität: 01.09.82 DE 3232515

(43) Veröffentlichungstag der Anmeldung:
07.03.84 Patentblatt 84/10

(45) Bekanntmachung des Hinweises auf die
Patenterteilung:
10.06.87 Patentblatt 87/24

(45) Bekanntmachung des Hinweises auf die
Entscheidung über den Einspruch:
21.08.91 Patentblatt 91/34

(84) Benannte Vertragsstaaten:
AT CH DE FR LI NL

(56) Entgegenhaltungen:
EP-A- 0 009 672
EP-A- 0 010 136
EP-A- 0 040 416
WO-A-80/02795
WO-A-81/02831
DE-A- 2 203 942
DE-A- 2 911 601

(56) Entgegenhaltungen:
DE-A- 2 930 663
DE-A- 3 026 643
US-A- 4 197 853
US-A- 4 324 256
Acta anaest. scand.1978, Suppl.68, H.N.Yeung
et al "Low Impedance pH Sensitive Electrochemical Devices that are Potentially Applicable to Transcutaneous PCO2 Measurements
"; pp.137-141

(73) Patentinhaber: Hellige GmbH
Postfach 728 Heinrich-von-Stephan-Strasse 4
W-7800 Freiburg im Breisgau (DE)

(72) Erfinder: Ahsbahs, Walter
Alte Strasse 1
W-7802 Merzhausen (DE)
Erfinder: Hopmeier, Joachim, Dipl.-Ing.
Tiroler Weg 12
W-7800 Freiburg (DE)

(74) Vertreter: TER MEER - MÜLLER -
STEINMEISTER & PARTNER
Mauerkircherstrasse 45
W-8000 München 80 (DE)

EP 0 102 033 B2

**Beschreibung**

Die Erfindung betrifft einen elektrochemischen Sensor zur fortlaufenden, nichtinvasiven, transcutanen Überwachung des Partialdrucks von Kohlendioxid im Blut oder Gewebe eines Menschen oder auch eines anderen Lebewesens, wobei als Meßverfahren die pH-Messung in einem Elektrolyten dient, der über eine elektrolytundurchlässige, kohlendioxiddurchlässige Membran mit einem Raum oder einer Fläche in Gasaustausch steht, dessen (deren) Kohlendioxid-Partialdruck gemessen werden soll.

Dieses Meßverfahren hat sich für die Messung des Kohlendioxid-Partialdrucks in Blutproben durchgesetzt und ist in diesem Zusammenhang in Lehrbüchern vielfach beschrieben, so z.B. im Buch "Säure-Basen-Haushalt und Blutgase" von O. Müller-Plathe, 2. Aufl., Stuttgart-New York 1982, S. 174 ff. Es wird hierbei als Stand der Technik vorausgesetzt, die pH-Messung elektrochemisch mit der für diesen Zweck bekannten Glaselektrode in geeigneter Ausführungsform durchzuführen.

Aus der Druckschrift US-A-4 197 853 ist ein elektrochemischer Sensor zur gleichzeitigen und transcutanen oder intra-arteriellen Messung des Sauerstoffpartialdrucks $pO_2$ und des Kohlendioxid-partialdrucks $pCO_2$ des Blutes bekannt. Zur transcutanen $pCO_2$-Messung ist aus der Druckschrift DE-A-29 11 343 ein ähnlicher Sensor in verschiedenen Ausführungen bekannt. Diese bekannten Sensoren weisen als $CO_2$-Nachweiselement eine pH-Glaselektrode auf und sind zur Hyperämisierung der Meßstelle auf eine konstante Temperatur oberhalb der normalen Körpertemperatur beheizt und thermostatisiert.

Aus der DE-A-20 33 584 ist es ebenfalls bekannt, das Meßverfahren für eine nichtinvasive Messung des Kohlendioxid-Partialdrucks durch die Haut hindurch, also eine transcutane Messung, unter Verwendung einer Glaselektrode anzuwenden.

Die bekannte Glaselektrode ist in der pH-Meßtechnik zwar bewährt und weit verbreitet ; sie hat jedoch für den genannten Anwendungsfall erhebliche Nachteile, die darauf zurückzuführen sind, daß der Kohlendioxid-Sensor, der auf der Haut befestigt wird, möglichst klein sein soll und mit einer möglichst dünnen und flexiblen Leitung mit dem anzeigenden Meßgerät verbunden werden muß. Ein wichtiger Gesichtspunkt ist weiterhin, daß der Sensor sich problemlos im klinischen Routinebetrieb, etwa auf einer Intensivstation, handhaben lassen und unempfindlich gegen gelegentliche Fehlbehandlung wie Druck, Stoß und Schlag sein soll. Auch sollen der Versand und die Aufbewahrung nicht benutzter Sensoren keine besonderen Schwierigkeiten bereiten.

Diesen Forderungen wird die Glaselektrode nicht gerecht. in den erforderlichen kleinen Abmessungen ist sie schwierig herzustellen und sie hat einen sehr hohen elektrischen Innenwiderstand in der Größenordnung von etwa 1 000 MΩ, der zum Einbau eines Vorverstärkers (Impedanzwandlers) in den Sensor oder zur Benutzung einer für den vorgesehenen Anwendungsfall unpraktischen Art einer geschirmten Leitung zwischen dem Sensor und dem Meßgerät zwingt, um elektrische Störungen zu vermeiden. Auch ist die dünne Meßmembran mechanisch leicht verletzlich und empfindlich gegen Austrocknung. Der Elektrolyt der kleinen Glaselektrode begrenzt infolge seiner geringen Menge auch deren Lebensdauer und macht den Sensor frostempfindlich, was insbesondere beim Versand nachteilig ist.

Auf dem Hintergrund der beschriebenen Nachteile der Glaselektrode liegt der Erfindung die Aufgabe zugrunde, einen Sensor zur transcutanen $pCO_2$-Messung zu schaffen, der bei guter Ansprechempfindlichkeit räumlich sehr klein, insbesondere sehr flach hergestellt werden kann, sich durch einen vergleichsweise niedrigen Innenwiderstand auszeichnet, und der überdies weitgehend unempfindlich gegen Stoß und Schlag ist und sich problemlos über lange Zeiträume lagern läßt.

Ein erfindungsgemäßer elektrochemischer Sensor weist die im Patentanspruch 1 angegebenen Merkmale auf.

Vorteilhafte Weiterbildungen des erfindungsgemäßen Sensors sind in Unteransprüchen gekennzeichnet.

Als Vorstufe zur Erfindung wurde zunächst nach anderen geeigneten Verfahren zur Messung des pH-Werts gesucht, wobei in diesem speziellen Fall die Besonderheit in Betracht gezogen werden mußte, daß der pH-Wert immer nur im gleichen Medium des Elektrolyten gemessen wird.

Im Lehrbuch für Elektrochemie von Kortüm, 4. Auflage, Weinheim/Bergstraße 1966, S. 314 bis 316, ist eine Reihe von Möglichkeiten angegeben pH-Messungen mit Metall/Metalloxid-Elektroden durchzuführen. Bereits in der DE-PS 701 788 aus dem Jahre 1941 ist auch die Verwendung speziell behandelter Metall-Elektroden, z.B. einer Antimon-Antimonoxid-Elektrode zur Messung des pH-Werts des Bluts vorgeschlagen worden. Und schließlich ist es aus einer Veröffentlichung aus dem Jahre 1976, "Electrochemical Sensor for Continuous Transcutaneous Measurement", Journal Applied Physiol., Band 41, S. 442 bis 447, durch einen Aufsatz von Beran, Huxtable, Sperling bekannt, eine Antimon/Antimonoxid-Elektrode als pH-sensitives Element für einen transcutan zu verwendenden $pCO_2$-Sensor einzusetzen.

Wegen der unter anderem von Kortüm (s.o.) erwähnten Empfindlichkeit der Metalloxid-Elektroden auch gegenüber dem Sauerstoff-Partialdruck, der beim Einsatz des Sensors zur Messung an Lebewesen immer auch vorhanden ist, konnten sich die bisher bekannt gewordenen Lösungen mit Metalloxid-Elektroden für den hier vorgesehenen Zweck nicht durchsetzen. In dem erwähnten Buch von Kortüm wird auch darauf hingewiesen, daß außer Antimon Metalle wie Fe, W, Sn, Zn, Ir, Os, Sg und Cu unter gewissen Bedingungen in einem Elektrolyten ein Potential zeigen, das linear vom pH-Wert abhängt. Dabei wird jedoch Sauerstoff-Freiheit des Elektrolyten vorausgesetzt - eine Bedingung, die in der Praxis des Routinebetriebs kaum zu erfüllen ist.

Neuere, in Acta anaesth. scand. 1978, Suppl. 68, 137-141 von Hong N. YEUNG et al. unter dem Titel "Low Impedance pH Sensitive Electrochemical Devices that are Potentially Applicable to Transcutaneous PCO$_2$-Measurements" beschriebene Untersuchungen wenden sich speziell dem Problem der Verringerung der Sauerstoffempfindlichkeit zu. So wird u.a. die Wahl von Metallen vorgeschlagen, die sich durch niedrige Austauschströme bei der Sauerstoffreduktion auszeichnen. Als Beispiele werden Palladium und Iridium genannt. Die Untersuchungen schließen mit der Feststellung, daß keiner der beschriebenen Sensoren die Glaselektrode ersetzen könne. Empfehlungen gehen dahin, eine sogenannte Pellet-Elektrode auf der Basis Pd-PdO$_2$ weiter zu untersuchen oder die wissenschaftliche Aufmerksamkeit einer oberflächenpassivierten Antimon-Einkristallelektrode zuzuwenden.

Es wurde nun gefunden, daß überraschenderweise das Potential einer Metallelektrode aus Iridium/Iridiumoxid in einem Elektrolyten zumindest bei Sauerstoffpartialdrücken, wie sie bei der vorgesehenen Anwendung vorkommen, d.h. unter I bar, vom Sauerstoff-Partialdruck in der Elektrolyt-Lösung unbeeinflußt bleibt. Durch die Kombination der Iridium/ Iridiumoxid-Elektrode mit einer Referenzelektrode insbesondere aus Silber/Silberchlorid könnte ein Sensor zur transcutanen pCO$_2$-Messung verwirklicht werden, der sehr vorteilhafte Eigenschaften hat. Dieser Sensor ist insbesondere weitgehend unempfindlich gegen Sauerstoff und Narkosegase, mechanisch widerstandsfähig, unempfindlich gegenüber Fehlbehandlung durch Stoß oder Schlag sowie gegen Austrocknen ; er zeichnet sich überdies in der vorgeschlagenen Form durch einen Innenwiderstand von nur ca. IOO kΩ aus, so daß das Meßsignal ohne Vorverstärker im Sensor störungsfrei zum Meßgerät geleitet werden kann. Das Meßsignal ist im pH-Bereich 5,5 bis 8.5 linear vom pH-Wert abhängig und entspricht annähernd dem theoretischen NERNST-Potential.

Der erfindungsgemäße Sensor läßt sich außerdem sehr flach herstellen, was für die Anwendung zur Überwachung von Patienten sehr erwünscht ist.

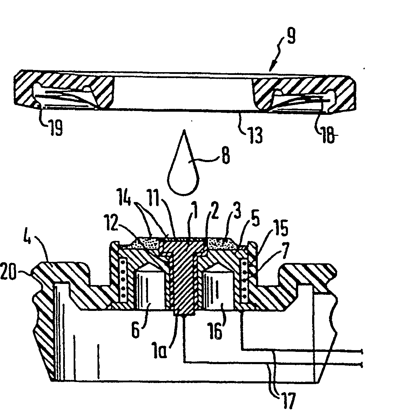

Die Erfindung und vorteilhafte Einzelheiten werden nachfolgend unter Bezug auf die Zeichnung in einer beispielsweisen Ausführungsform näher erläutert.

Die einzige Figur zeigt in schematischer Darstellung das Schnittbild eines Ausführungsbeispiels des erfindungsgemäßen pH-Wert-Sensors mit einer konzentrischen Elektrodenanordnung.

Die Zeichnung läßt ein ringförmiges Gehäuse 4 aus Kunststoff erkennen mit einem mittigen Durchbruch I5, in den eine Silber-Silberchlorid-Referenzelektrode 3 paßgenau eingesetzt ist, die eine zentrale Bohrung I2 aufweist, in die in konzentrischer Anordnung eine Iridium/Iridiumoxid-Elektrode I von beispielsweise 2 mm Durchmesser eingesteckt ist, die durch eine Kunststoff-Gießharzschicht 2 gegen die Referenzelektrode 3 isoliert ist. Die Ir/IrOx-Elektrode I könnte aus einem zylinderförmigen einheitlichen Iridiumstück hergestellt sein, das oberflächenseitig, also auf der einer Membran I3 zugekehrten und in einen Elektrolyt 8 als Pufferlösung eintauchenden Oberfläche oxidiert ist, was durch elektrochemische oder thermochemische Verfahren geschehen kann. Da Iridium jedoch ein vergleichsweise sehr teures Metall ist, wird es aus Preisgründen in der Regel vorteilhaft sein, den wie in der Zeichnung dargestellt beispielsweise pilzförmig gestalteten Körper der Ir/IrOx-Elektrode I stirnseitig, d.h. wiederum auf der in den Elektrolyten 8 eintauchenden Oberfläche mit einem Iridiumplättchen II zu versehen, das mit dem übrigen Elektrodenkörper Ia, der beispielsweise aus Silber oder Kupfer bestehen kann, elektrisch und thermisch gut leitend verbunden ist. Die in den Elektrolyten 8 eintauchende Oberfläche des Iridiumplättchens II ist dann wiederum elektrochemisch oder thermochemisch oxidiert, wobei das Oxid auch in hydratisierter Form als Iridium-Oxidhydrat vorliegen kann. Solche Oxidationsverfahren sind beispielsweise in Gmelin, Handbuch der anorganischen Chemie, Band Iridium, 1939, S. 44 ff. sowie im "Rapport Technique RT 62 of CEBEL-COR, Chapter I, Section 13.5, Iridium, S. 377, sowie in der Veröffentlichung "Cyclic Voltametric Studies on Iridium Electrodes in Sulphuric Acid Solutions" von RAND und WOODS in Electroanalytical Chem. interfac. Electrochem. 55 (1974), S. 375 bis 381 beschrieben.

Die Referenzelektrode 3 ist entweder durch eine zumindest teilweise chlorierte Oberfläche des auf eine konstante Temperatur zwischen 37°C und 45°C aufheizbaren Silberkörpers 5 gebildet oder als ringförmiges Plättchen aus Silber/Silberchlorid-Sintermetall auf den Silberkörper 5 aufplattiert. Der Silberkörper 5 ist mit einer ringförmig umlaufenden breiten Nut versehen, in die eine Heizwicklung 7 eingesetzt ist. Die Heizleistungszufuhr zum Silberkörper wird über einen in eine exzentrische Bohrung 6 eingesetzten Temperaturfühler (nicht dargestellt), beispielsweise einen Thermistor geregelt. In eine weitere, ebenfalls exzentrisch zur Ir/IrOx-Elektrode I angeordnete Bohrung I6 ist ein weiterer Temperaturfühler (ebenfalls nicht gezeigt) wiederum beispielsweise ein Thermistor eingesetzt, der ein Schalterelement zur unmittelbaren Unterbrechung der Heizleistungszufuhr zur Heizwicklung 7 unter

bricht, wenn die Temperatur des Silberkörpers 5 einen vorgebbaren Schwellenwert von beispielsweise 42°C überschreitet. Der von dem in die Bohrung I6 eingesetzten Thermistor gesteuerte elektronische Schalter kann unmittelbar in das Gehäuse 4 des Sensors eingebaut und direkt aus der Speiseleitung für die Heizwicklung 7 mit Strom versorgt sein. Das Meßsignal wird als Potentialdifferenz zwischen der Ir/IrOx-Elektrode I und der Referenzelektrode 3, 5 über eine bei I7 angedeutete Leitung abgegriffen.

Um den Sensor für einen Meßvorgang vorzubereiten, wird, wie die Zeichnung erkennen läßt, ein kleiner Tropfen des Elektrolyten 8 auf die durchgehende Oberfläche I4 der Referenzelektrode 3 und der Ir/IrOx-Elektrode I aufgebracht. Sodann wird der Sensor mit einer vergefertigten einmal zu benutzenden Schnappring-Membranvorrichtung 9 bespannt, wie sie beispielsweise in der DE-OS 30 40 544 beschrieben ist. Die Befestigung des Sensors auf der Haut erfolgt in bekannter Weise mit einem doppelseitigen Klebering I8. Derwie in der Zeichnung erkennbar leicht gewölbt gefertigte Schnappring 9 weist eine nach innen vorspringende umlaufende Rastkante I9 auf. Wird der Schnappring auf das Gehäuse 4 aufgedrückt, so rastet die Rastkante I9 hinter dem vorspringenden Rand 2O am Gehäuse 4 ein und die Membran I3 wird einwandfrei zentriert über die Oberfläche I4 gespannt unter Zwischenschaltung einer dünnen Schicht des Elektrolyten 8.

## Patentansprüche

1. Elektrochemischer Sensor zur transcutanen Messung des Kohlendioxid-Partialdrucks ($pCO_2$) eines Lebewesens, der auf dem Prinzip der $pCO_2$-Messung durch Messung des pH-Werts eines Elektrolyten beruht, der über eine $CO_2$-durchlässige, jedoch für den Elektrolyten undurchlässige Membran mit der Meßstelle im Gasaustausch steht und der zur Hyperämisierung der Meßstelle auf eine konstante Temperatur zwischen 37°C und 45°C beheizt und thermostatisiert ist, wobei eine mit dem Elektrolyten in Berührungskontakt stehende Meßelektrode (I) vorhanden ist, deren Potential gegen eine ebenfalls mit dem Elektrolyten in Berührung stehende Referenzelektrode (3) abgreifbar ist und bei dem die Meßelektrode (I) aus einem gut wärmeleitenden Elektrodenkörper (Ia) besteht, auf dessen im wesentlichen flacher, der Membran zugekehrter Stirnfläche ein Iridiumplättchen (II) leitend befestigt ist, das auf der mit dem Elektrolyten in Berührung stehenden freien Oberfläche eine Iridiumoxidschicht aufweist.

2. Sensor nach Anspruch I, dadurch gekennzeichnet, daß die Iridiumschicht (II) durch elektrochemische und/oder thermochemische Umwandlung der Oberfläche des Iridiumplättchens (II) hergestellt ist.

3. Sensor nach Anspruch I, dadurch gekennzeichnet, daß der Elektrodenkörper (Ia) pilzförmig gestaltet, mit einer Isolierschicht (2) umgeben und in eine angepaßte zentrische Bohrung (I2) der Referenzelektrode (3) eingesetzt ist.

4. Sensor nach Anspruch I, dadurch gekennzeichnet, daß der Elektrodenkörper (Ia) Zylinderform aufweist, mit einer Isolierschicht umgeben und in eine zentrische Bohrung (I2) der Referenzelektrode (3) eingesetzt ist.

5. Sensor nach einem der vorstehenden Ansprüche, dadurch gekennzeichnet, daß die Referenzelektrode (3) mit einer Heizwicklung (7) umgeben ist und Bohrungen (6, I6) aufweist, in die Temperaturfühler eingesetzt sind.

6. Sensor nach einem der vorstehenden Ansprüche, dadurch gekennzeichnet, daß die Referenzelektrode (3) aus Silber besteht und auf der der Membran (I3) zugekehrten Oberfläche eine Silberchloridschicht (I4) aufweist.

7. Sensor nach einem der vorstehenden Ansprüche I bis 6, dadurch gekennzeichnet, daß die Referenzelektrode (3) auf der der Membran (I3) zugekehrten Oberfläche eine Schicht aus Ag/AgCl-Sintermetall aufweist.

8. Sensor nach Anspruch 5, dadurch gekennzeichnet, daß in eine Bohrung (6) ein Temperaturfühler eingesetzt ist, der in Verbindung mit einer Regelschaltung den Strom durch die Heizwicklung (7) in der Weise steuert, daß der Sensor auf gleichbleibender Temperatur gehalten wird, und in eine Bohrung (I6) ein weiterer Temperaturfühler eingesetzt ist, der bei Erreichen eines einstellbaren oberen Temperaturwerts des Sensors den Heizstrom unterbricht.

## Claims

1. An electrochemical sensor for the transcutaneous measurement of the partial pressure of carbon dioxide ($pCO_2$) of an organism, based on the principle of $pCO_2$ measurement by measuring the pH value of an electrolyte which is in gas exchange with the measuring point through a diaphragm permeable to $CO_2$ but impermeable for the electrolyte and which is heated, for the hyeremisation of the measuring point, to a constant temperature between 37 and 45°C and is thermostatically controlled, wherein a measuring electrode (I) is provided which is in physical contact with the electrolyte and whose potential can be picked off relative to a reference electrode (3) which is also in contact with the electrolyte, and wherein said measuring electrode (I) consists of an electrode body (Ia) of high heat conductivity and that an iridium platelet (II) is conductively fixed to the substantially flat front face of said electrode which faces said diaphragm, said platelet (II) having an iridium oxide layer on the free surface which is in contact with said elec-

trolyte.

2. The sensor of claim I, **characterized in that** the iridium oxide layer (II) is prepared by electrochemical and/or thermochemical changing the surface of the iridium platelet (II).

3. The sensor of claim I, **characterized in that** said electrode body (Ia) is mushroom-shaped, surrounded by an insulating layer (2) and inserted into a matching central bore (I2) of said reference electrode (3).

4. The sensor of claim I, **characterized in that** said electrode body (Ia) is cylindrical, surrounded by an insulating layer and inserted into a central bore (I2) of the reference electrode (3).

5. The sensor of anyone of the preceding claims **characterized in that** said reference electrode (3) is surrounded by a heating coil (7) and has bores (6, I6) into which temperature sensors are inserted.

6. The sensor of anyone of the preceding claims, **characterized in that** said reference electrode (3) is made of silver and has a silver chloride layer (I4) on the surface facing said diaphragm (I3).

7. The sensor of anyone of the preceding claims I to 6, **characterized in that** said reference electrode (3) has a layer of Ag/AgCl sintered metal on the surface facing said diaphragm (I3).

8. The sensor of claim 6, **characterized in that** a temperature sensor is inserted in a bore (6) which in combination with a control circuit controls the flow of current through said heating coil (7) in such a manner that the sensor is kept at a constant temperature, and that an additional temperature sensor is inserted in a bore (I6) which interrupts the heating current when an adjustable upper temperature value of the sensor is reached.

## Revendications

1. Capteur électrochimique pour la mesure transcutanée de la pression partielle du gaz carbonique (pCO$_2$) d'un être vivant qui repose sur le principe de la mesure de la valeur pH d'un électrolyte qui, par l'intermédiaire d'une membrane perméable au CO$_2$ mais imperméable à l'électrolyte, réalise un échange de gaz avec le point de mesure et qui, pour l'hyperémisation du point de mesure, est chauffé et thermostaté à une température constante comprise entre 37°C et 45°C, ledit capteur comprenant une électrode de mesure (I) dont le potentiel peut être prélevé par rapport à une électrode de référence (3) également en contact avec l'électrolyte et cette électrode de mesure (I) est constituée par un corps d'électrode (Ia) bon conducteur de chaleur dont la face frontale essentiellement plate et dirigée vers la membrane est munie, en connexion conductrice, d'une plaquette d'iridium (II) dont la surface libre en contact avec l'électrolyte est recouverte d'une couche d'oxyde d'iridium.

2. Capteur selon la revendication I, **caractérisé en ce que** la couche d'oxyde d'iridium (II) est réalisée par transformation électrochimique et/ou thermochimique de la surface de la plaque d'iridium (II).

3. Capteur selon la revendication I, **caractérisé en ce que** le corps (Ia) de l'électrode présente une forme de champignon, qu'il est entouré d'une couche isolante (2) et qu'il est engagé dans un alésage ajusté central (I2) de l'électrode de référence (3).

4. Capteur selon la revendication I, **caractérisé en ce que** le corps (Ia) de l'électrode présente une forme cylindrique, qu'il est entouré d'une couche isolante et qu'il est engagé dans un alésage central (I2) de l'électrode de référence (3).

5. Capteur selon l'une quelconque des revendications I à 4, **caractérisé en ce que** l'électrode de référence (3) est entourée d'un enroulement chauffant (7) et qu'elle présente des alésages (6, I6) dans lesquels sont engagés des sondes de température.

6. Capteur selon l'une quelconque des revendications I à 5, **caractérisé en ce que** l'électrode de référence (3) est réalisée en argent et que sa surface dirigée vers la membrane (I3) est munie d'une couche de chlorure d'argent (I4).

7. Capteur selon l'une quelconque des revendications I à 6, **caractérisé en ce que** l'électrode de référence (3) présente à sa surface dirigée vers la membrane (I3) une couche de métal fritté Ag/AgCl.

8. Capteur selon la revendication 5, **caractérisé en ce que** dans un alésage (6) est engagée une sonde de température qui, en combinaison avec un circuit de réglage, commande le courant envoyé dans l'enroulement chauffant (7) de telle façon que le capteur est maintenu à une température constante, et que dans un alésage (I6) est engagée une autre sonde de température qui coupe le courant de chauffage dés l'atteinte d'une valeur de température supérieure réglable du capteur.